# EUROPÄISCHE PATENTSCHRIFT

(11) **EP 2 007 310 B1**
(45) Veröffentlichungstag und Bekanntmachung des Hinweises auf die Patenterteilung: **12.08.2015**
(21) Anmeldenummer: 07718374.7
(22) Anmeldetag: 06.04.2007
(51) Int. Cl.: A61F 2/00

(54) **IMPLANTIERBARES NETZ FÜR EINE CHIRURGISCHE REKONSTRUKTION IM BEREICH DES BECKENBODENS**
IMPLANTABLE MESH FOR SURGICAL RECONSTRUCTION IN THE AREA OF THE PELVIC FLOOR
RÉSEAU IMPLANTABLE POUR UNE RECONSTRUCTION CHIRURGICALE DANS LA ZONE DU PLANCHER PELVIEN

(30) Priorität: 14.04.2006 AT 6502006
(43) Veröffentlichungstag der Anmeldung: 31.12.2008
(73) Patentinhaber: A.M.I. Agency for Medical Innovations GmbH, 6800 Feldkirch (AT)
(72) Erfinder: FARNSWORTH, Bruce, St. Ives 2015 (AU)
(74) Vertreter: Hofmann, Ralf U.
(86) Internationale Anmeldenummer: PCT/AT2007/000157
(87) Internationale Veröffentlichungsnummer: WO 2007/118260

(56) Entgegenhaltungen:
- EP-A- 1 600 118
- WO-A1-03/096929
- WO-A1-2007/081954
- WO-A1-2007/109508
- WO-A2-2007/106897
- FR-A1- 2 852 818
- US-A1- 2006 058 575

## Beschreibung

Die Erfindung betrifft ein implantierbares Netz für eine chirurgische Rekonstruktion im Bereich des Beckenbodens, mit einem anterioren Netzabschnitt zur Anordnung zwischen der Blase und der Vagina, einem posterioren Netzabschnitt zur Anordnung zwischen der Vagina und dem Rektum, einem vom anterioren Netzabschnitt ausgehenden Paar von distalen Transobturator-Haltearmen zur ausgehend vom anterioren Netzabschnitt nach lateral und ventral verlaufenden Platzierung durch den Obturator, einem vom anterioren Netzabschnitt ausgehenden Paar von proximalen Transobturator-Haltearmen zur ausgehend vom anterioren Netzabschnitt nach lateral und ventral verlaufenden Platzierung durch den Obturator und einem vom posterioren Netzabschnitt ausgehenden Paar von unteren dorsalen Haltearmen zur ausgehend vom posterioren Netzabschnitt nach lateral und dorsal verlaufenden Platzierung.

Implantierbare Netze für chirurgische Rekonstruktionen im Bereich des weiblichen Beckenbodens sind bekannt. Eine anteriore Wiederherstellung (Rekonstruktion), bei der das Netz im Bereich zwischen der Blase und der Vagina implantiert wird, wird insbesondere bei einem Blasenvorfall (=einer Zystocele) durchgeführt. Eine posteriore Wiederherstellung (Rekonstruktion), bei der das Netz zwischen der Vagina und dem Rektum implantiert wird, wird insbesondere bei einem Mastdarmvorfall (=einer Rektocele) durchgeführt. Bei einer vollständigen Wiederherstellung werden eine anteriore und eine posteriore Wiederherstellung kombiniert, wobei üblicherweise bei entferntem Uterus ein durchgehendes Netz mit einem anterioren Netzabschnitt und einem posterioren Netzabschnitt eingesetzt wird.

Ein bekanntes Netz zur Durchführung einer anterioren und posterioren Wiederherstellung weist Haltearme zur Halterung des Netzes an Körperstrukturen auf, die jeweils paarweise von den beiden Seiten des Netzes ausgehen. Hierbei gehen vom anterioren Netzabschnitt zwei Paare von Obturator-Haltearmen aus. Diese verlaufen im implantierten Zustand des Netzes ausgehend vom anterioren Netzabschnitt nach lateral und ventral und erstrecken sich durch den Transobturator und werden im Weiteren unterhalb des Schambeins durch die Haut herausgeführt. An diesen aus der Haut herausgeführten Abschnitten kann bei der Operation nach dem Einsetzen des Netzes eine entsprechende Zugkraft ausgeübt werden, um das Netz richtig zu platzieren. Die aus der Haut herausstehenden Abschnitte werden dann vor dem Vernähen der Haut abgeschnitten. Weiters besitzt der posteriore Netzabschnitt ein Paar von dorsalen Haltearmen, die bei der Operation ausgehend vom posterioren Netzabschnitt nach lateral und dorsal platziert werden und entweder mit dem Sakrospinalband vernäht werden oder durch das Sakrospinalband durchgeführt und Richtung ventral umgelenkt werden und im Weiteren durch den Transobdurator und die Haut im Bereich unterhalb des Schambeins geführt werden, um nach der Ausrichtung des Netzes entsprechend abgeschnitten zu werden.

Durch dieses vorbekannte implantierbare Netz für eine vollständige Wiederherstellung, wird noch keine optimale Abstützung der zu rekonstruierenden Körperstrukturen erreicht. Insbesondere besteht eine Gefahr einer Verkürzung im Bereich der proximalen Vagina und es kann auch zu Schmerzen und einer Dysperennia kommen. Weiters ist auch die posteriore Abstützung gegen einen Rektumvorfall nicht optimal.

Wenn von diesem herkömmlichen Netz Teile abgeschnitten werden, so dass entweder nur mehr im Wesentlichen der anteriore Netzabschnitt oder nur mehr im Wesentlichen der posteriore Netzabschnitt übrig bleibt, so kann mit diesem herkömmlichen Netz auch eine nur anteriore Rekonstruktion oder eine nur posteriore Rekonstruktion durchgeführt werden. Auch hierbei ist die Abstützung der Körperstrukturen nicht optimal.

Aufgabe der Erfindung ist es, ein implantierbares Netz der eingangs genannten Art bereitzustellen, durch welches eine verbesserte Rekonstruktion ermöglicht wird. Erfindungsgemäß gelingt dies durch ein Netz mit den Merkmalen des Anspruchs 1.

Durch ein erfindungsgemäßes implantierbares Netz gelingt bei der Durchführung einer vollständigen Rekonstruktion eine natürlichere Rekonstruktion der proximalen Vagina. Hierbei liegt der Zwischenabschnitt des Netzes vor dem proximalen Ende der Vagina und durch die beidseitig des Zwischenabschnitts an diesen angrenzenden Paare von dorsalen Haltearmen kann eine gute Ausrichtung und Halterung des Netzes erreicht werden.

Der posteriore Netzabschnitt ist zusätzlich mit Translevator-Haltearmen versehen. Diese können bei der Implantation des Netzes nach lateral und ventral verlaufend durch den Levator-Muskel geführt werden und im Anschluss daran durch die Haut herausgeführt werden, wobei eine entsprechende Zugspannung zur Einrichtung des Netzes ausübbar ist. Nach der Operation können diese Haltearme unterhalb der Haut entsprechend abgeschnitten werden, worauf die Haut vernäht wird. Hierbei ist zumindest ein Paar von proximalen Translevator-Haltearmen und ein Paar von distalen Translevator-Haltearmen vorhanden, die weiter proximal und weiter distal durch den Levator-Muskel geführt werden.

Wenn im Rahmen dieser Schrift von "proximal" und "distal" die Rede ist, so ist dies jeweils auf die Lage zum Uterus bezogen, d.h. ein proximales Teil liegt näher beim Uterus als ein distales Teil.

In einer vorteilhaften Ausführungsform der Erfindung ist der Zwischenabschnitt des Netzes mit beidseitig abstehenden Lappen versehen. Diese können bei der Operation an die lateralen Wände der proximalen Vagina angelegt werden, um eine Abstützung gegen ein Eindrücken von benachbarten Strukturen zu erreichen.

Weitere Vorteile und Einzelheiten der Erfindung werden im Folgenden anhand der beiliegenden Zeichnung erläutert. In dieser zeigen:
- Fig.: 1 eine stark schematisierte, nicht maßstäbliche Darstellung eines implantierten Netzes gemäß dem Stand der Technik für eine vollständige chirurgische Rekonstruktion;
- Fig. 2: eine Darstellung entsprechend Fig. 1 für ein erfindungsgemäßes Netz;
- Fig. 3: eine Darstellung entsprechend Fig. 2, gemäß einer modifizierten Anbringung des erfindungsgemäßen Netzes;
- Fig. 4: eine Ansicht eines erfindungsgemäßen Netzes;
- Fig. 5: eine stark schematisierte, nicht maßstäbliche Darstellung des implantierten Netzes, in einem Schnitt im Bereich zwischen Blase und Vagina, wobei der anteriore Abschnitt des Netzes sichtbar ist;
- Fig. 6: eine stark schematisierte, nicht maßstäbliche Darstellung des implantierten Netzes, in einem Schnitt im Bereich zwischen der Vagina und dem Rektum, wobei der posteriore Abschnitt des Netzes sichtbar ist;
- Fig. 7: eine Schrägsicht eines chirurgischen Instruments für das Einziehen eines Haltearms;
- Fig. 8: eine perspektivische Darstellung des Endabschnitts des Instruments mit einem Endabschnitt des Haltearms des Netzes mit einem Verbindungselement zur Verbindung mit dem Instrument;
- Fig. 9: einen gegenüberliegenden Endabschnitt des Instruments mit einem Endabschnitt eines ankoppelbaren weiteren medizinischen Instruments;
- Fig. 10: ein weiteres Ausführungsbeispiel eines medizinischen Instruments zum Einziehen eines Haltearms;
- Fig. 11: einen Endabschnitt dieses Instruments mit einem Endabschnitt des Haltearms des Netzes mit einem angepassten Verbindungselement;
- Fig. 12: den gegenüberliegenden Endabschnitt des Instruments angekoppelt an ein weiteres medizinisches Instrument;
- Fig. 13: eine weitere Ausführungsform eines medizinischen Instruments zum Einziehen eines Haltearms;
- Fig. 14: einen Endabschnitt des Instruments und einen Endabschnitt eines Haltearms;
- Fig. 15: einen gegenüberliegenden Endabschnitt des Instruments angekoppelt an ein weiteres medizinisches Instrument;
- Fig. 16: einen Endabschnitt eines Instruments zum Einziehen eines Haltearms gemäß einer weiteren Ausführungsvariante mit einem Endabschnitt des Haltearms, der ein angepasstes Verbindungselement aufweist.

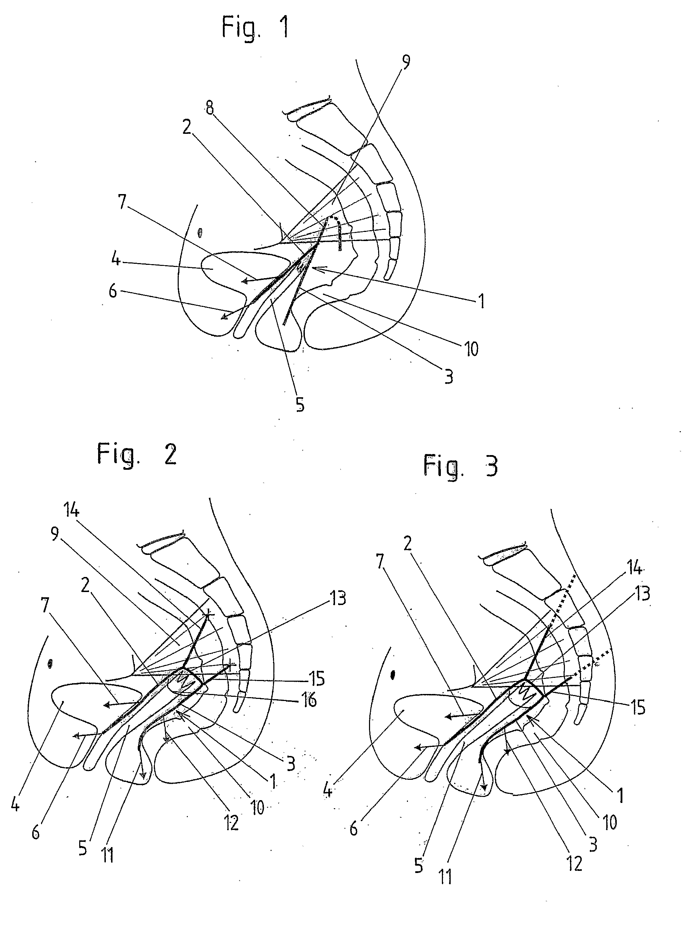

In Fig. 1 ist schematisch eine vollständige chirurgische Rekonstruktion im Bereich des Beckenbodens mit einem implantierbaren Netz gemäß dem Stand der Technik dargestellt.

Das Netz 1 umfasst einen anterioren Netzabschnitt 2, der zwischen der Blase 4 und der Vagina 5 angeordnet ist und einen posterioren Netzabschnitt 3, der zwischen der Vagina 5 und dem Rektum 10 angeordnet ist. Der anteriore Netzabschnitt 2 ist mit einem Paar von distalen Transobturator-Haltearmen 6 und einem Paar von proximalen Transobturator-Haltearmen 7 ausgestattet, welche jeweils ausgehend vom anterioren Netzabschnitt 2 nach lateral und ventral verlaufen. Diese Haltearme und ihre Verläufe sind in Fig. 1 lediglich durch Pfeile schematisch angedeutet. Diese Haltearme 6, 7 werden bei der Implantation des Netzes durch den Obturator geführt.

Der posteriore Netzabschnitt 3 ist mit einem Paar von dorsalen Haltearmen 8 ausgestattet, die ausgehend vom proximalen Ende des posterioren Netzabschnitts 2 nach lateral und dorsal verlaufen und durch das Sakrospinalband 9 durchgeführt sind. Es ergibt sich dadurch eine in Seitenansicht gesehen insgesamt etwa V-förmige Anordnung des anterioren und posterioren Netzabschnitts 2, 3.

Fig. 2 zeigt stark schematisiert die Platzierung eines erfindungsgemäßen Netzes bei einer vollständigen chirurgischen Konstruktion im Bereich des Beckenbodens, wobei der Uterus entfernt ist. Das Netz umfasst wiederum einen anterioren Netzabschnitt 2, der zwischen der Blase 4 und der Vagina 5 angeordnet ist und einen posterioren Netzabschnitt 3, der zwischen der Vagina 5 und dem Rektum 10 angeordnet ist. Vom anterioren Netzabschnitt 2 geht ein Paar von distalen Transobturator-Haltearmen 6 sowie ein Paar von proximalen Transobturator-Haltearmen 7 aus, die jeweils nach lateral und ventral verlaufen und durch den Obturator geführt sind. Diese Transobturator-Haltearme 6, 7 sind wiederum nur durch Pfeile angedeutet.

Vom posterioren Netzabschnitt 3 geht ein Paar von distalen Translevator-Haltearmen 11 sowie ein Paar von proximalen Translevator-Haltearmen 12 aus. Diese verlaufen ausgehend vom posterioren Netzabschnitt 3 nach lateral und ventral und sind durch den Levator-Muskel geführt. Diese Translevator-Haltearme 11, 12 sind in Fig. 2 nur durch Pfeile schematisch angedeutet.

Das Netz weist zwischen dem anterioren Netzabschnitt 2 und dem posterioren Netzabschnitt 3 einen Zwischenabschnitt 13 auf. Vom an den Zwischenabschnitt 13 angrenzenden proximalen Ende des anterioren Netzabschnitts 2 geht ein Paar von oberen dorsalen Haltearmen 14 aus. Diese verlaufen ausgehend vom anterioren Netzabschnitt 2 nach lateral und dorsal und sind an das Sakrospinalband 9 angenäht. Stattdessen könnten sie auch durch das Sakrospinalband 9 durchgeführt werden, im weiteren Verlauf nach ventral umgelenkt sein und unterhalb des Schambeins durch die Haut durchgeführt sein, wobei nach der Operation der aus der Haut herausstehende Teil abgeschnitten wird.

Vom an den Zwischenabschnitt 13 angrenzenden proximalen Ende des posterioren Netzabschnitts 3 geht ein Paar von unteren dorsalen Haltearmen 15 aus. Diese verlaufen ausgehend vom posterioren Netzabschnitt 3 nach lateral und dorsal und sind am Sakrospinalband 9 angenäht. Stattdessen könnten sie auch durch das Sakrospinalband 9 durchgeführt sein, in der Folge nach ventral umgelenkt sein und unterhalb des Schambelns aus der Haut herausgeführt sein, wobei nach der Operation der aus der Haut herausstehende Teil abgeschnitten wird.

Bei einem erfindungsgemäßen Netz 1 ergibt sich in Seitenansicht gesehen nach der Operation eine annähernd U-förmige Anordnung des anterioren Netzabschnitts 2, Zwischenabschnitts 13 und posterioren Netzabschnitts 3, wie dies aus Fig. 2 sichtbar ist. Die Rekonstruktion im Bereich der proximalen Vagina 5 wird dadurch wesentlich verbessert, weiters wird durch die Translevator-Haltearme 11, 12 die Halterung des posterioren Netzabschnitts 3 wesentlich verbessert.

In einer bevorzugten Ausführungsvariante der Erfindung sind am Zwischenabschnitt 13 beidseitig abstehende Lappen 16 angeordnet, die in Fig. 2 lediglich durch eine Linie angedeutet sind und an die lateralen Wände der proximalen Vagina angelegt sind, wodurch eine verbesserte Abstützung dieses Bereichs der Vagina gegen ein Eindrücken von benachbarten Körperstrukturen erreicht wird.

Fig. 3 zeigt stark schematisiert eine etwas modifizierte Anbringung eines erfindungsgemäßen Netzes 1. Die oberen und unteren dorsalen Haltearme 14, 15 werden hier nach lateral und dorsal durch den Kokzygeal-Muskel oder das Sakro-Tuberus-Band geführt (welche in Fig. 3 der Übersichtlichkeit halber nicht dargestellt sind) und im Weiteren nach dorsal und durch die Haut geführt, wobei nach der Operation der aus der Haut herausstehende Teil abgeschnitten wird. Auch bei dieser Befestigung ergibt sich eine im Wesentlichen U-förmige Anordnung des anterioren Netzabschnitts 2, Zwischenabschnitts 13 und posterioren Netzabschnitts 3.

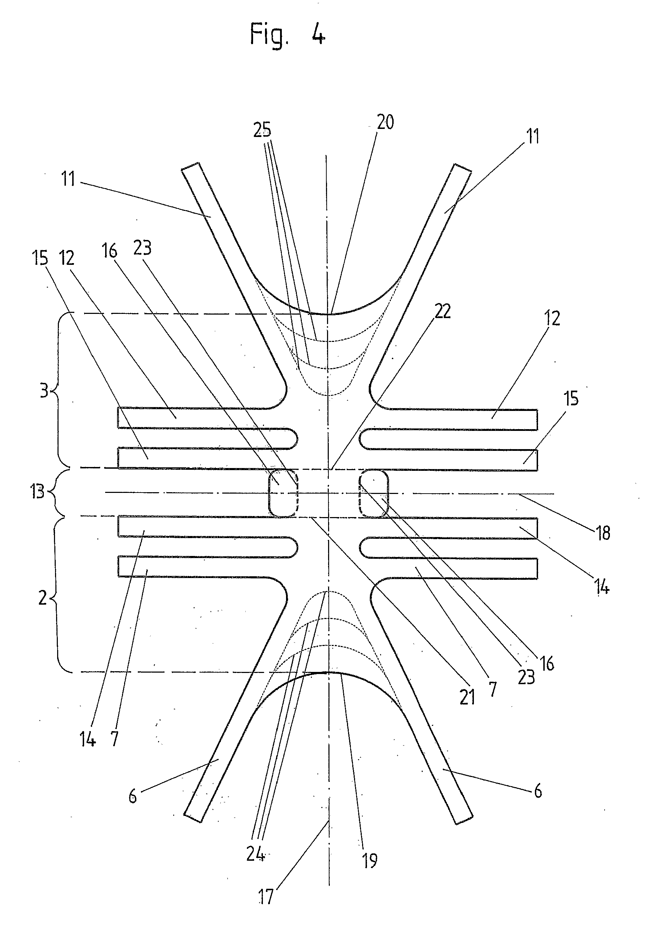

Eine vorteilhafte Ausführungsvariante eines erfindungsgemäßen Netzes ist in Fig. 4 in größerem Detail dargestellt. Das Netz ist bezüglich einer Längsmittellinie 17 spiegelsymmetrisch ausgebildet, wobei die beiden Haltearme 6, 7, 11, 12, 14, 15 eines jeweiligen Paars spiegelsymmetrisch zueinander liegen.

In der gezeigten Ausführungsvariante ist das Netz weiters bezüglich einer den Zwischenabschnitt 13 mittig durchsetzenden Quermittellinie 18 spiegelsymmetrisch ausgebildet. Eine nicht-symmetrische Ausbildung bezüglich der Quermittellinie 18 ist denkbar und möglich, um eine weitere Anpassung des Netzes an die Körperstrukturen zu ermöglichen.

Vom anterioren Netzabschnitt 2 gehen, wie bereits erwähnt, ein Paar distaler Transobturator-Haltearme 6 und ein Paar proximaler Transobturator-Haltearme 7 aus. Beispielsweise gehen die distalen Transobturator-Haltearme 6 vom distalen Ende 19 des anterioren Netzabschnitts 2 unter einem Winkel zwischen 20° bis 60° zur Längsmittellinie 17 aus. Die proximalen Transobturator-Haltearme 7 gehen beispielsweise von einem mittleren Bereich des anterioren Netzabschnitts 2 unter einem Winkel von im Wesentlichen 90° (d.h. Abweichungen von ± 20° sollen erfasst sein) zur Längsmittellinie 17 aus.

Vom anterioren Netzabschnitt 2 geht weiters ein Paar von oberen dorsalen Haltearmen 14 aus, und zwar von demjenigen Teil des anterioren Netzabschnitts 2, der an den Zwischenabschnitt 13 angrenzt. Beispielsweise gehen die oberen dorsalen Haltearme 14 unter einem Winkel von im Wesentlichen 90° (d.h. eine Abweichung von ± 20° soll erfasst sein) zur Längsmittellinie 17 aus.

Vom posterioren Netzabschnitt 3 geht ein Paar von unteren dorsalen Haltearmen 15 aus, und zwar von demjenigen Teil des posterioren Netzabschnitts 3, der an den Zwischenabschnitt 13 angrenzt. Beispielsweise gehen die unteren dorsalen Haltearme 15 vom posterioren Netzabschnitt unter einem Winkel von im Wesentlichen 90° (d.h. Abweichungen von je ± 20° sollen erfasst sein) zur Längsmittellinie 17 aus.

Vom posterioren Netzabschnitt gehen weiters Translevator-Haltearme 11, 12 aus. Diese gehen von einem Teil des posterioren Netzabschnitts 3 aus, der weiter beim distalen Ende 20 des posterioren Netzabschnitts 3 liegt. Es ist ein Paar von distalen Translevator-Haltearmen 11 und ein Paar von proximalen Translevator-Haltearmen 12 vorhanden. Die distalen Translevator-Haltearme 11 gehen vom distalen Ende 20 des posterioren Netzabschnitts 3 aus, wobei sie beispielsweise einen Winkel im Bereich zwischen 20° bis 60° mit der Längsmittellinie 17 einschließen. Die proximalen Translevator-Haltearme 12 gehen von einem mittleren Bereich des posterioren Netzabschnitts 3 aus, wobei sie beispielsweise einen Winkel von im Wesentlichen 90° (d.h. eine Abweichung von ± 20° soll erfasst sein) zur Längsmittellinie 17 einschließen.

Der zwischen den Stellen, von denen die oberen und unteren dorsalen Haltearme 14, 15 ausgehen, gelegene Zwischenabschnitt 13 weist eine wesentlich geringere Erstreckung in Richtung der Längsmittellinie 17 auf als der anteriore und der posteriore Netzabschnitt 2, 3. Bei der Implantation des Netzes wird der Zwischenabschnitt 13 gegenüber dem anterioren Netzabschnitt 2 und der posteriore Netzabschnitt 3 gegenüber dem Zwischenabschnitt 13 in die gleiche Richtung umgeknickt bzw. umgebogen. Entsprechende Biegelinien 21, 22, die parallel zur Quermittellinie 18 liegen, sind als punktierte Linien dargestellt. In der Praxis wird es eher zu einer Umbiegung mit einem gewissen Radius als zu einer scharfen Abknickung kommen. Die Umbiegung zwischen dem anterioren Netzabschnitt 2 und dem Zwischenabschnitt 13 sowie dem Zwischenabschnitt 13 und dem posterioren Netzabschnitt 3 liegt jeweils im Bereich von im Wesentlichen 90° (d.h. Abweichungen von je ± 20° sollen erfasst sein).

Am Zwischenabschnitt 13 sind beidseitig seitlich abstehende Lappen 16 angeordnet. Diese werden bei der Implantation zur Anlage an die lateralen Wände der proximalen Vagina um parallel zur Längsmittellinie 17 liegende Biegelinien 23 umgebogen, die in Fig. 4 durch punktierte Linien dargestellt sind. Wiederum wird im implantierten Zustand eher eine Umbiegung mit einem gewissen Radius als ein scharfer Knick vorliegen.

Im Bereich der distalen Enden 19, 20 des anterioren und posterioren Netzabschnitts 2, 3 sind Markierungen 24, 25 auf das Netz 1 aufgebracht. Dadurch wird ein intraoperatives Zurechtschneiden des Netzes 1 erleichtert, um das Netz 1 an den jeweiligen Patienten anzupassen. Solche Markierungen können beispielsweise auch im Bereich der Lappen 16 aufgebracht sein.

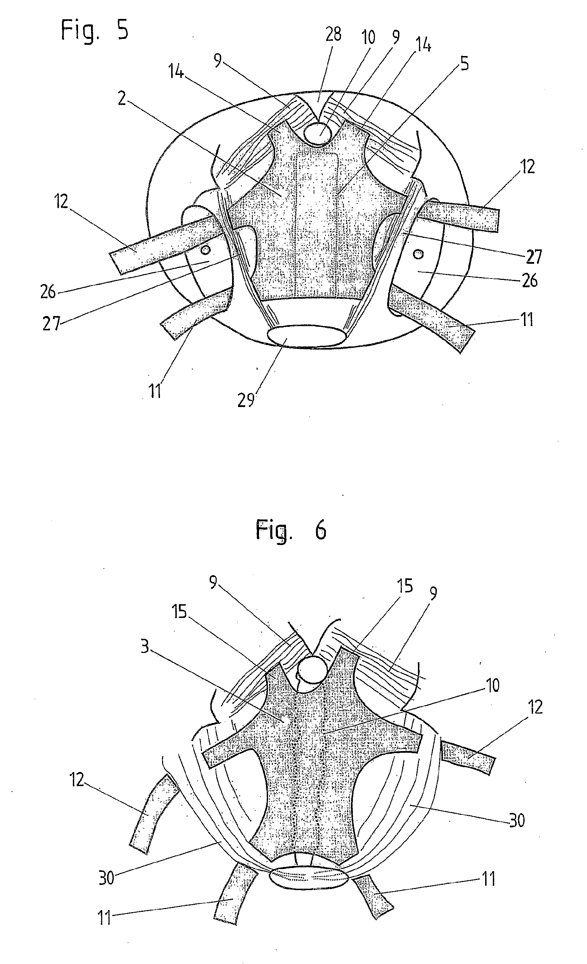

Die Fig. 5 und 6 zeigen schematisierte Darstellungen der Anordnung des anterioren und posterioren Netzabschnitts 2, 3 im implantierten Zustand des Netzes 1 aus einem gegenüber Fig. 2 unterschiedlichen Blickwinkel (jeweils etwa in Draufsicht auf den entsprechenden Netzabschnitt). Die Netzabschnitte 2, 3 sind in den schematischen Darstellungen von Fig. 5 und 6 gegenüber Fig. 4 etwas unterschiedlich dargestellt (insbesondere in ihren Verhältnissen Länge zu Breite), die Netzabschnitte 2, 3 können aber die in Fig. 4 dargestellte Gestalt aufweisen.

Aus Fig. 5 ist ersichtlich, wie die Transobturator-Haltearme 6, 7 durch den jeweiligen Obturator 26 geführt sind. Sie können hierbei durch die Faszie "Arcus tendium fasciae pelvis" 27 geführt sein. Die oberen dorsalen Haltearme 14 sind am jeweiligen Sakrospinalband 9 festgenäht. In Fig. 5 sind weiters das Sakrum 28, das Schambein 29, das Rektum 10 und die unterhalb des anterioren Netzabschnitts 2 liegende Vagina 5 (punktiert) angedeutet.

Aus Fig. 6 ist die Durchführung der Translevator-Haltearme 11, 12 durch den jeweiligen Levator-Muskel 30 ersichtlich, wobei die distalen Translevator-Haltearme 11 weiter distal als die proximalen Translevator-Haltearme 12 durch den jeweiligen Levator-Muskel 30 geführt sind. Weiters ist in Fig. 6 angedeutet, dass die unteren dorsalen Haltearme 15 am jeweiligen Sakrospinalband 9 festgenäht sind.

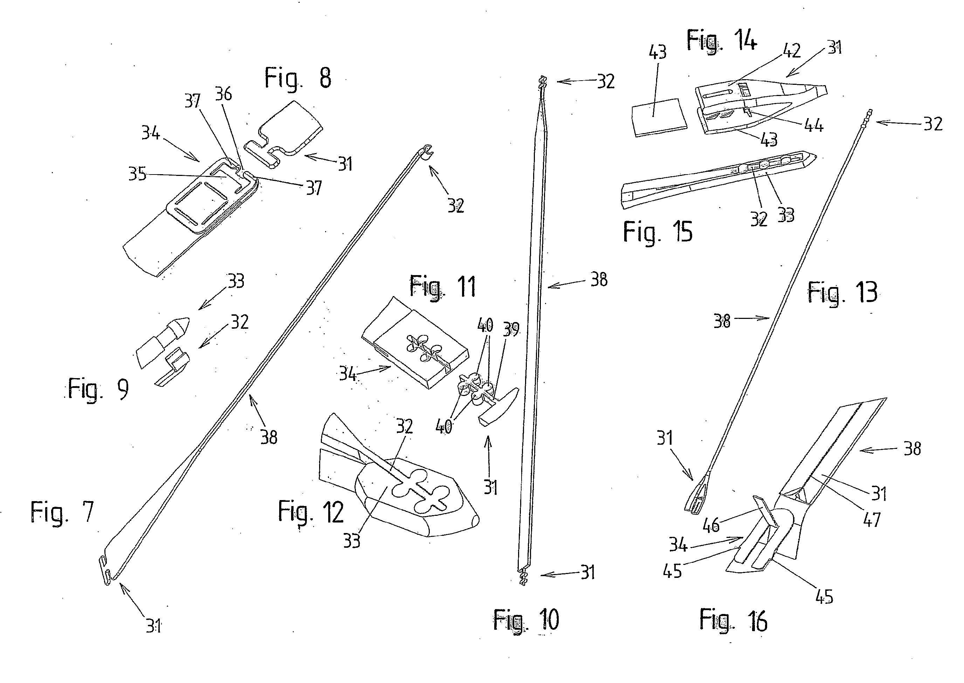

Ein medizinisches Instrument 38 zum Einziehen eines Haltearms durch einen Gewebekanal ist in Fig. 7 dargestellt. Das Instrument 38 besitzt einen T-förmigen Verbindungsabschnitt 31 zum Einhängen des freien Endes des Haltearms. Zum Verbindungsabschnitt 31 hin verbreitet sich das Instrument 38 bis auf die Breite des Haltearms, wobei es eine geringe Dicke aufweist. Am anderen Ende ist ein Koppelteil 32 zur Ankopplung an ein weiteres medizinisches Instrument vorgesehen, von dem in Fig. 9 ein Koppelabschnitt 33 dargestellt ist.

Der Haltearm besitzt endseitig ein Verbindungselement 34 (vgl. Fig. 8). Dieses wird im gezeigten Ausführungsbeispiel von einem Metallstück gebildet, in welches der T-förmige Verbindungsabschnitt 31 einhängbar ist. Im eingehängten Zustand sichert eine federelastische Lasche 35 den Verbindungsabschnitt 31 in seinem eingehängten Zustand. Zum Einhängen des Verbindungsabschnitts 31 weist das Verbindungselement 34 beidseitig einer Öffnung 36 U-förmige Abschnitte 37 auf, in welche der T-Steg des Verbindungsabschnitts 31 einhängbar ist.

Anstelle der Anbringung eines Verbindungselements 34 am Ende des Haltearms könnte auch das Ende des Haltearms umgeschlagen und vernäht sein, wodurch eine Schlaufe ausgebildet wird. Vom Ende her könnte in diese Schlaufe ein Schlitz eingebracht sein, so dass sich beidseitig des Schlitzes Einhängeschlaufen ergeben, in welche der T-Steg des Verbindungsabschnitts 31 einhängbar ist.

Mittels eines derartigen medizinischen Instruments 38 kann ein jeweiliger Haltearm des Netzes 1 faltenfrei in das Gewebe eingezogen werden. Bei der Implantation des Netzes wird vorzugsweise eine der Anzahl der Haltearme entsprechende Anzahl von Instrumenten 38 in den Körper eingeführt. In der Folge werden die Haltearme an die Verbindungsabschnitte 31 der Instrumente 38 angekoppelt und im Weiteren werden die Haltearme mittels der Instrumente 38 durch das Gewebe durchgezogen.

Eine weitere Ausführungsvariante eines Instruments 38 zum Einziehen eines Haltearms ist in Fig. 10 dargestellt. Der Verbindungsabschnitt 31 des Instruments 38 weist hier einen nach vorne abstehenden Steg 39 mit seitlichen Fortsätzen 40 auf (vgl. Fig. 11). Das Verbindungselement 34 am Ende des Haltearms ist mit einer Vertiefung versehen, deren Form dem Verbindungsabschnitt 31 entspricht, um ein Ankoppeln des Verbindungsabschnitts 31 am Verbindungselement 34 zu ermöglichen.

Das Instrument 38 kann am anderen Ende wiederum mittels eines Koppelteils 32 an einen Koppelabschnitt 33 eines weiteren medizinischen Instruments angekoppelt werden, mit dem das Instrument 38 manipuliert werden kann. Diese Ankopplung kann beispielsweise in analoger Weise wie die Ankopplung an den Haltearm ausgebildet sein (vgl. Fig. 12).

Das medizinische Instrument 38 gemäß dieser Ausführungsvariante weist wiederum anschließend an den Verbindungsabschnitt 31 eine dem Haltearm im Wesentlichen entsprechende Form auf (in Breite und Dicke), die hier über den Großteil der Länge des Instruments fortgeführt ist.

Eine weitere Ausführungsform eines medizinischen Instruments 38 zum Einziehen eines Haltearms ist in Fig. 13 dargestellt. Der Verbindungsabschnitt 31, der vergrößert in Fig. 14 dargestellt ist, ist hier zangenförmig ausgebildet. Zwischen die beiden Zangenteile 41, 42 ist das Ende 43 des Haltearms einführbar und in der Folge fixierbar, indem die Zangenteile 41, 42 zusammengedrückt und mittels eines Rastelements 44 in der zusammengedrückten Position verrastet werden. Am Ende des Haltearms muss bei dieser Ausführungsform kein spezielles Verbindungselement angeordnet werden.

Am anderen Ende des Instruments ist wiederum ein Koppelteil 32 zur Ankopplung an einen Koppelabschnitt 33 eines weiteren medizinischen Instruments vorgesehen (vgl. Fig. 15).

Fig. 16 zeigt eine weitere Möglichkeit der Verbindung eines Haltearms mit einem medizinischen Instrument zum Einziehen des Haltearms. Das medizinische Instrument 38 besitzt hier zumindest im Bereich eines endseitigen Verbindungsabschnitts 31 einen nach vorne offenen Einführkanal, der in Form eines flachen Rechtecks ausgebildet ist. Am Ende des Haltearms ist ein Verbindungselement 34 angebracht. Dieses weist ein flaches Metallstück mit seitlich vorragenden sägezahnförmigen Spitzen 45 auf. Wenn das Verbindungselement 34 in den Einführkanal des Verbindungsabschnitts 31 eingeführt ist, so wirken diese Spitzen 45 einem Herausziehen des Verbindungselements 34 entgegen. Weiters weist das Verbindungselement 34 eine von der Ebene des flachen Metallteils abstehende federelastische Lasche 46 auf, die ebenfalls zur Sicherung des Verbindungselements 34 im Verbindungsabschnitt 31 dient. Wenn das Verbindungselement 34 wiederum aus dem Verbindungsabschnitt 31 entfernt werden soll, so wird hierzu der Verbindungsabschnitt 31 entlang einer vorgegebenen Aufreißlinie 47 aufgerissen.

Das Netz besteht aus einem körperverträglichen Material, in welches Körpergewebe einwachsen kann. Beispielsweise besteht das Netz aus Polypropylen.

### Legende

| zu den Hinweisziffern: | | | |
|---|---|---|---|
| 1 | Netz | 25 | Markierung |
| 2 | anteriorer Netzabschnitt | 26 | Obturator |
| 3 | posteriorer Netzabschnitt | 27 | Faszie |
| 4 | Blase | 28 | Sakrum |
| 5 | Vagina | 29 | Schambein |
| 6 | distaler Transobturator-Haltearm | 30 | Levator-Muskel |
| 7 | proximaler Transobturator-Haltearm | 31 | Verbindungsabschnitt |
| 8 | dorsaler Haltearm | 32 | Koppelteil |
| 9 | Sakrospinalband | 33 | Koppelabschnitt |
| 10 | Rektum | 34 | Verbindungselement |
| 11 | distaler Translevator-Haltearm | 35 | Lasche |
| 12 | proximaler Translevator-Haltearm | 36 | Öffnung |
| 13 | Zwischenabschnitt | 37 | Abschnitt |
| 14 | oberer dorsaler Haltearm | 38 | Instrument |
| 15 | unterer dorsaler Haltearm | 39 | Steg |
| 16 | Lappen | 40 | Fortsatz |
| 17 | Längsmittellinie | 41 | Zangenteil |
| 18 | Quermittellinie | 42 | Zangenteil |
| 19 | distales Ende | 43 | Ende |
| 20 | distales Ende | 44 | Rastelement |
| 21 | Biegelinie | 45 | Spitze |
| 22 | Biegelinie | 46 | Lasche |
| 23 | Biegelinie | 47 | Aufreißlinie |
| 24 | Markierung | | |

## Patentansprüche

1. Implantierbares Netz für eine chirurgische Rekonstruktion im Bereich des Beckenbodens, mit einem anterioren Netzabschnitt (2) zur Anordnung zwischen der Blase (4) und der Vagina (5), einem posterioren Netzabschnitt (3) zur Anordnung zwischen der Vagina (5) und dem Rektum (10), einem vom anterioren Netzabschnitt (2) ausgehenden Paar von distalen Transobturator-Haltearmen (6) zur ausgehend vom anterioren Netzabschnitt (2) nach lateral und ventral verlaufenden Platzierung durch den Obturator (26), einem vom anterioren Netzabschnitt (2) ausgehenden Paar von proximalen Transobturator-Haltearmen (7) zur ausgehend vom anterioren Netzabschnitt (2) nach lateral und ventral verlaufenden Platzierung durch den Obturator (26) und einem vom posterioren Netzabschnitt (3) ausgehenden Paar von unteren dorsalen Haltearmen (15) zur ausgehend vom posterioren Netzabschnitt (3) nach lateral und dorsal verlaufenden Platzierung, **dadurch gekennzeichnet, dass** das Netz einen zwischen dem anterioren Netzabschnitt (2) und dem posterioren Netzabschnitt (3) liegenden Zwischenabschnitt (13) aufweist, wobei das Paar von unteren dorsalen Haltearmen (15) im an den Zwischenabschnitt (13) angrenzenden Bereich des posterioren Netzabschnitts (3) ausgeht, und dass weiters im an den Zwischenabschnitt (13) angrenzenden Bereich des anterioren Netzabschnitts (2) ein Paar von oberen dorsalen Haltearmen (14) zur ausgehend vom anterioren Netzabschnitt (2) nach lateral und dorsal verlaufenden Platzierung und vom posterioren Netzabschnitt (3) ein Paar von distalen Translevator-Haltearmen (11) und ein Paar von proximalen Translevator-Haltearmen (12) zur ausgehend vom posterioren Netzabschnitt (3) nach lateral und ventral verlaufenden Platzierung durch den Levator-Muskel (30) ausgehen.

2. Implantierbares Netz nach Anspruch 1, **dadurch gekennzeichnet, dass** das Netz bezüglich einer die Abschnitte (2, 3, 13) des Netzes mittig durchsetzenden Längsmittellinie (17) spiegelsymmetrisch ausgebildet ist.

3. Implantierbares Netz nach Anspruch 2, **dadurch gekennzeichnet, dass** das Netz bezüglich einer den Zwischenabschnitt (13) mittig durchsetzenden Quermittellinie (18), die rechtwinklig zur Längsmittellinie (17) liegt, spiegelsymmetrisch ausgebildet ist.

4. Implantierbares Netz nach Anspruch 2 oder Anspruch 3, **dadurch gekennzeichnet, dass** die distalen Transobturator-Haltearme (6) von einem distalen Ende (19) des anterioren Netzabschnitts (2) unter einem Winkel zwischen 20° bis 60° zur Längsmittellinie (17) ausgehen.

5. Implantierbares Netz nach einem der Ansprüche 2 bis 4, **dadurch gekennzeichnet, dass** die proximalen Transobturator-Haltearme (6) von einem mittleren Bereich des anterioren Netzabschnitts (2) unter einem Winkel von im Wesentlichen 90° zur Längsmittellinie (17) ausgehen.

6. Implantierbares Netz nach einem der Ansprüche 2 bis 5, **dadurch gekennzeichnet, dass** der obere dorsale Haltearm (14) vom anterioren Netzabschnitt (2) unter einem Winkel von im Wesentlichen 90° zur Längsmittellinie (17) ausgeht.

7. Implantierbares Netz nach einem der Ansprüche 2 bis 6, **dadurch gekennzeichnet, dass** der untere dorsale Haltearm (15) vom posterioren Netzabschnitt (3) unter einem Winkel von im Wesentlichen 90° zur Längsmittellinie (17) ausgeht.

8. Implantierbares Netz nach einem der Ansprüche 1 bis 7, **dadurch gekennzeichnet, dass** die distalen Translevator-Haltearme (11) von einem distalen Ende (20) des posterioren Netzabschnitts (3) unter einem Winkel zwischen 20° bis 60° zur Längsmittellinie (17) ausgehen.

9. Implantierbares Netz nach einem der Ansprüche 1 bis 8, **dadurch gekennzeichnet, dass** die proximalen Translevator-Haltearme (12) von einem mittleren Bereich des posterioren Netzabschnitts (3) unter einem Winkel von im Wesentlichen 90° zur Längsmittellinie (17) ausgehen.

10. Implantierbares Netz nach einem der Ansprüche 1 bis 9, **dadurch gekennzeichnet, dass** der Zwischenabschnitt (13) beidseitig abstehende Lappen (16) zum Anlegen an laterale Wände der proximalen Vagina (5) aufweist.

## Claims

1. Implantable mesh for surgical reconstruction in the area of the pelvic floor, comprising an anterior mesh portion (2) for arrangement between the bladder (4) and the vagina (5), a posterior mesh portion (3) for arrangement between the vagina (5) and the rectum (10), a pair of distal transobturator holding arms (6) originating from the anterior mesh portion (2) for placement extending laterally and ventrally originating from the anterior mesh portion (2) through the obturator (26), a pair of proximal transobturator holding arms (7) originating from the anterior mesh portion (2) for placement extending laterally and ventrally originating from the anterior mesh portion (2) through the obturator (26), and a pair of lower dorsal holding arms (15) originating from the posterior mesh portion (3) for placement extending laterally and dorsally originating from the posterior mesh portion (3), **characterised in that** the mesh comprises an intermediate portion (13) lying between the anterior mesh portion (2) and the posterior mesh portion (3), wherein the pair of lower dorsal holding arms (15) originates in the region of the posterior mesh portion (3) adjoining the intermediate portion (13), and **in that** furthermore in the region of the anterior mesh portion (2) adjoining the intermediate portion (13), there originate a pair of upper dorsal holding arms (14) for placement extending laterally and dorsally originating from the anterior mesh portion (2), and from the posterior mesh portion (3), a pair of distal transelevator holding arms (11), and a pair of proximal transelevator holding arms (12) for placement extending laterally and ventrally originating from the posterior mesh portion (3) through the levator muscle (30).

2. Implantable mesh according to claim 1, **characterised in that** the mesh is designed mirrorsymmetrically relative to a longitudinal centre line (17) passing centrally through one of the portions (2, 3, 13) of the mesh.

3. Implantable mesh according to claim 2, **characterised in that** the mesh is designed mirrorsymmetrically relative to a transverse centre line (18) passing centrally through the intermediate portion (13) and lying at right angles to the longitudinal centre line (17).

4. Implantable mesh according to claim 2 or 3, **characterised in that** the distal transobturator holding arms (6) originate from a distal end (19) of the anterior mesh portion (2) at an angle of between 20° and 60° to the longitudinal centre line (17).

5. Implantable mesh according to one of the claims 2 to 4, **characterised in that** the proximal transobturator holding arms (6) originate from the central region of the anterior mesh portion (2) at an angle of essentially 90° to the longitudinal centre line (17).

6. Implantable mesh according to one of the claims 2 to 5, **characterised in that** the upper dorsal holding arm (14) originates from the anterior mesh portion (2) at an angle of essentially 90° to the longitudinal centre line (17).

7. Implantable mesh according to one of the claims 2 to 6, **characterised in that** the lower dorsal holding arm (15) originates from the posterior mesh portion (3) at an angle of essentially 90° to the longitudinal centre line (17).

8. Implantable mesh according to one of the claims 1 to 7, **characterised in that** the distal transelevator holding arms (11) originate from a distal end (20) of the posterior mesh portion (3) at an angle of between 20° and 60° to the longitudinal centre line (17).

9. Implantable mesh according to one of the claims 1 to 8, **characterised in that** the proximal transelevator holding arms (12) originate from a central region of the posterior mesh portion (3) at an angle of essentially 90° to the longitudinal centre line (17).

10. Implantable mesh according to one of the claims 1 to 9, **characterised in that** the intermediate portion (13) comprises bilaterally projecting flaps (16) for placement on lateral walls of the proximal vagina (5).

## Revendications

1. Bandelette en tissu implantable destinée à permettre une reconstruction chirurgicale dans la zone du plancher pelvien comprenant un segment de bandelette antérieur (2) destiné à être positionné entre la vessie (4) et le vagin (5), un segment de bandelette postérieur (3) destiné à être positionné entre le vagin (5) et le rectum (10),
une paire de bras de maintien du transobturateur distaux (6) partant du segment de bandelette antérieur (2) et destinés à être mis en place au travers de l'obturateur (26) à partir du segment de bandelette antérieur (2) après un déplacement latéral et ventral, une paire de bras de maintien du transobturateur proximaux (7) partant du segment de bandelette antérieur (2) et destinés à être mis en place au travers de l'obturateur (26) à partir du segment de bandelette antérieur (3) après un déplacement latéral et ventral, et une paire de bras de maintien dorsaux inférieurs (15) partant du segment de bandelette postérieur (3) destinés à être mis en place en partant du segment de bandelette postérieur après un déplacement latéral et dorsal,
**caractérisé en ce que**
la bandelette comporte un segment intermédiaire (13) situé entre le segment de bandelette antérieur (2) et le segment de bandelette postérieur (3), la paire de bras de maintien dorsaux inférieurs (15) partant de la zone du segment de bandelette postérieur (3) voisine du segment intermédiaire (13), et il est en outre prévu, dans la zone du segment de bandelette antérieur (2) voisine du segment intermédiaire (13) une paire de bras de maintien dorsaux supérieurs (14) destinés à être mis en place en partant du segment de bandelette antérieur (2) après un déplacement latéral et dorsal, et, à partir du segment de bandelette postérieur (3) une paire de bras de maintien du transreleveur distaux (11) et une paire de bras de maintien du transreleveur proximaux (12) destinés à être mis en place au travers du muscle releveur (30) à partir du segment de bandelette postérieur (3) après un déplacement latéral et ventral.

2. Bandelette en tissu implantable conforme à la revendication 1,
**caractérisé en ce qu'**
elle présente une symétrie à miroir par rapport à un axe longitudinal médian (17) passant par le centre des segments (2, 3, 13).

3. Bandelette en tissu implantable conforme à la revendication 2,
**caractérisé en ce qu'**
elle présente une symétrie à miroir par rapport à un axe transversale médian (18) traversant le segment intermédiaire (13) en sa partie centrale et qui est perpendiculaire à l'axe longitudinal médian (17).

4. Bandelette en tissu implantable conforme à la revendication 2 ou à la revendication 3,
**caractérisé en ce que**
le bras de maintien du transobturateur distal (6) s'étend à partir de l'extrémité distale (19) du segment de bandelette antérieur (2) sous un angle compris entre 20° et 60°par rapport à l'axe longitudinal médian (17).

5. Bandelette en tissu implantable conforme à l'une des revendications 2 à 4,
**caractérisé en ce que**
le bras de maintien de l'obturateur transversal (6) proximal s'étend à partir de la zone médiane du segment de bandelette antérieur (2) sous un angle égal à essentiellement 90° par rapport à l'axe longitudinal médian (17).

6. Bandelette en tissu implantable conforme à l'une des revendications 2 à 5,
**caractérisé en ce que**
le bras de maintien dorsal supérieur (14) s'étend à partir du segment de bandelette antérieur (2) sous un angle égal à essentiellement 90° par rapport à l'axe longitudinal médian (17).

7. Bandelette en tissu implantable conforme à l'une des revendications 2 à 6,
**caractérisé en ce que**
le bras de maintien dorsal inférieur (15) s'étend à partir du segment de bandelette postérieur (3) sous un angle égal à essentiellement 90° par rapport à l'axe longitudinal médian (17).

8. Bandelette en tissu implantable conforme à l'une des revendications 1 à 7,
**caractérisé en ce que**
les bras de maintien du transreleveur distaux (11) s'étendent à partir de l'extrémité distale (20) du segment de bandelette postérieur (3) sous un angle compris entre 20° et 60° par rapport à l'axe longitudinal médian (17).

9. Bandelette en tissu implantable conforme à l'une des revendications 1 à 8,
**caractérisé en ce que**
les bras de maintien du transreleveur proximaux (12) s'étendent à partir de la zone médiane du segment de bandelette postérieur (3) sous un angle égal à essentiellement 90° par rapport à l'axe longitudinal médian (17).

10. Bandelette en tissu implantable conforme à l'une des revendications 1 à 9,
**caractérisé en ce que**
le segment intermédiaire (13) comprend de chaque côté des languettes s'écartant (16) destinées à s'appliquer sur les parois latérales du vagin proximal (5).
